(19) Europäisches Patentamt
European Patent Office
Office européen des brevets

(11) **EP 3 363 026 B1**

(12) **EUROPEAN PATENT SPECIFICATION**

(45) Date of publication and mention of the grant of the patent:
**16.08.2023 Bulletin 2023/33**

(21) Application number: **16856395.5**

(22) Date of filing: **17.10.2016**

(51) International Patent Classification (IPC):
*H01B 11/18* $^{(2006.01)}$     *A61B 18/12* $^{(2006.01)}$
*A61B 18/00* $^{(2006.01)}$     *A61B 18/04* $^{(2006.01)}$

(52) Cooperative Patent Classification (CPC):
**A61B 18/042;** A61B 2018/00166; A61B 2018/00178

(86) International application number:
**PCT/US2016/057310**

(87) International publication number:
**WO 2017/066745 (20.04.2017 Gazette 2017/16)**

(54) **LOW ELETROMAGNETIC FIELD ELECTROSURGICAL CABLE**

ELEKTROCHIRURGISCHES KABEL MIT NIEDRIGEM ELEKTROMAGNETISCHEM FELD

CÂBLE ÉLECTROCHIRURGICAL À FAIBLE CHAMP ÉLECTROMAGNÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.10.2015 US 201562242579 P**

(43) Date of publication of application:
**22.08.2018 Bulletin 2018/34**

(73) Proprietor: **U.S. Patent Innovations LLC**
**Takoma Park, MD 20912 (US)**

(72) Inventors:
• **CANADY, Jerome, M.D.**
  **Lakeland, FL 33803 (US)**
• **SHASHURIN, Alexey**
  **West Lafayette, IN 47906 (US)**
• **KEIDAR, Michael**
  **Baltimore, MD 21209 (US)**
• **ZHUANG, Taisen**
  **Takoma Park, MD 20912 (US)**

(74) Representative: **Tegethoff, Sebastian et al**
**Fortmann Tegethoff Patent- und Rechtsanwälte**
**Oranienburger Straße 39**
**10117 Berlin (DE)**

(56) References cited:
EP-A2- 1 832 243         WO-A1-2015/164676
GB-A- 2 052 836          US-A- 3 248 473
US-A- 4 092 485          US-A- 5 693 045
US-A1- 2006 180 111      US-A1- 2010 160 910
US-A1- 2013 296 846      US-B1- 6 190 385

## Description

## BACKGROUND OF THE INVENTION

Field of the Invention

The present invention relates to an electrosurgical cable which is not producing electromagnetic EM-field in its vicinity (zero-EM pollution) and reduces risk of electric shock for the human subjects involved in the electrosurgical procedure.

Background of the Related Art

Electrosurgical cables are used to connect an electrosurgical generator to an electrosurgical surgical hand piece and deliver high voltage and gas flow from the generator to the electrosurgical handpiece. Conventional electrosurgical cables utilized in electrosurgical systems consist of one high voltage electrode placed inside an electrically insulating flexible tube. The high voltage electrode inside the insulting tube creates strong electromagnetic (EM) field around the cable. Frequencies of the electrosurgical generators are below 1 MHz, which is associated with wavelengths $\lambda > 300$ m. Therefore, a conventional one-electrode electrosurgical cable effectively is a short antenna with length $L << \lambda$. Radiated EM power is low since antenna in far from the resonance; however, values of the electric field in the nearzone of the antenna are high due to high voltages applied to the electrode. Local electric fields can be as high as $E \sim V/D \sim 1000$ V/cm, taking very realistic separation between the electrosurgical cable and patient $D \sim 1$ cm, that can readily appear during the electrosurgical procedure when cable is constantly moving with respect to the patient. GB2052836 and EP1832243 are considered to be relevant prior art for the present invention.

## SUMMARY OF THE INVENTION

The present invention is disclosed in independent claim 1. A preferred embodiment is disclosed in the dependent claim 2.

The present invention relates to an electrosurgical cable that connects between an electrosurgical unit and a handpiece or housing that does not produce an EM-field or only a negligible EM-field in its vicinity. The cable can be used with any electrosurgical generator. The cable is intended to simultaneously deliver gas flow and high voltage electrical energy required for electrosurgical unit operation. Conventional electrosurgical cables utilized in the electrosurgical probes use only one conductor inside the insulating tube to which high voltage is applied. In contrast, present invention utilizes two conductors, namely an inner high voltage conductor and an outer conductor (connected to patient pad). The critical feature of present invention is that inner conductor electrical insulation that provides the following critical function.

tion. High voltage applied to the central electrode ($U_0$) is chosen above the breakdown threshold ($U_{BD}$) in order to initiate discharge on the electrosurgical handpiece ($U_0 > U_{BD}$). However, inside the cable a significant fraction of the applied voltage drops on the inner insulator, so that remaining voltage applied to the gas gap ($U_{gas}$) is below the breakdown threshold: $U_{gas} < U_{BD}$. This allows prevention of breakdown and ignition of plasma discharge inside the cable.

The present invention has two important benefits in comparison with conventional electrosurgical cables. First, the present cable is completely shielded and therefore it does not produce EM-field around itself in contrast with conventional electrosurgical probe cables which produce EM-field as regular short dipole antenna. Second, the present electrosurgical cable significantly reduces risk of electric shock of human subjects involved in electrosurgical procedure. Indeed, conventional electrosurgical cables can possess significant risk of electrical shock in case outer insulation layer is compromised. In contrast, compromising any insulators in the case when present invention is used may either cause human contact with shielded electrode or create short-circuit of the electrosurgical unit. Both events are electrically safe for the involved human subjects.

In a preferred embodiment the present invention is an electrosurgical cable having an elongated outer conductor, an outer insulator surrounding said outer conductor, said outer conductor and said outer insulator forming a tube, an elongated inner conductor inside said tube, and an inner insulator surrounding said inner conductor. There is a channel between and interior surface of said tube and said inner insulator. Further, sizes and materials of conductors and insulators are chosen so a voltage applied to the inner conductor is higher than the breakdown voltage and a voltage applied to gas flowing within said channel is below than the breakdown voltage. The electrosurgical cable may further have an electrical connector connected to said inner electrode for connecting said inner electrode to an electrosurgical power supply, an electrical connector connected to said out electrode for connecting said outer electrode to a ground, and a fluid connector connected to aid tube for connecting said tube to a fluid source.

The present invention is an electrosurgical cable having an elongated outer conductor having an outer radius $c$, an outer insulator surrounding said outer conductor and having inner radius $d$ and an outer radius $e$, said outer conductor and said outer insulator forming a tube, an elongated inner conductor inside said tube, said inner electrode having a radius $a$, an inner insulator surrounding said inner conductor, said inner insulator having an outer radius b. There is a channel between and interior surface of said tube and said inner insulator and the radii $a, b, c, d, e$ are selected so $a < b < c \leq d \leq e$. Further, the radii a, b, c, d and e are selected so a total applied voltage ($U_0$) is distributed between the inner insulator ($U_{in}$) and gas gap between inner and outer insulators ($U_{gas}$), so

that $U_0 = U_{in} + U_{gas}$. Still further, $a$, $b$, $c$, $d$ and $e$ may be selected so that $U_{in} \approx U_{gas}$. For example, the radii may be selected as follows: $a$=0.25 mm, $b$=2.5 mm, $c$=$d$=4 mm and $e$=5 mm.

**BRIEF DESCRIPTION OF THE FIGURES**

For a more complete understanding of the present invention and the advantages thereof, reference is now made to the following description and the accompanying drawings, in which:

> FIG. 1 is a perspective view of an electrosurgical cable in accordance with a preferred embodiment of the present invention with connectors on one end of the cable and an electrosurgical handpiece on the other end of the cable.
> FIG. 2 is a perspective view of a portion of a cable in accordance with a preferred embodiment of the present invention.
> FIG. 3 is a cross-section of a cable in accordance with a preferred embodiment of the present invention showing relationships of dimensions and voltage drops of various component parts of the cable.

**DETAILED DESCRIPTION OF THE PREFERRED EMBODIMENT**

In describing a preferred embodiment of the invention illustrated in the drawings specific terminology will be resorted to for the sake of clarity. However, the invention is not intended to be limited to the specific terms so selected, and it is to be understood that each specific term includes all technical equivalents that operate in similar manner to accomplish a similar purpose. The preferred embodiment of the invention is described for illustrative purposes, it being understood that the invention may be embodied in other forms not specifically shown in the drawings.

The present invention presents a novel concept of an electrosurgical cable which produces no EM-field or only negligible EM-field around itself (zero-EM pollution) and offers operation without risk of electric shock for human subjects involved in the electrosurgical procedure.

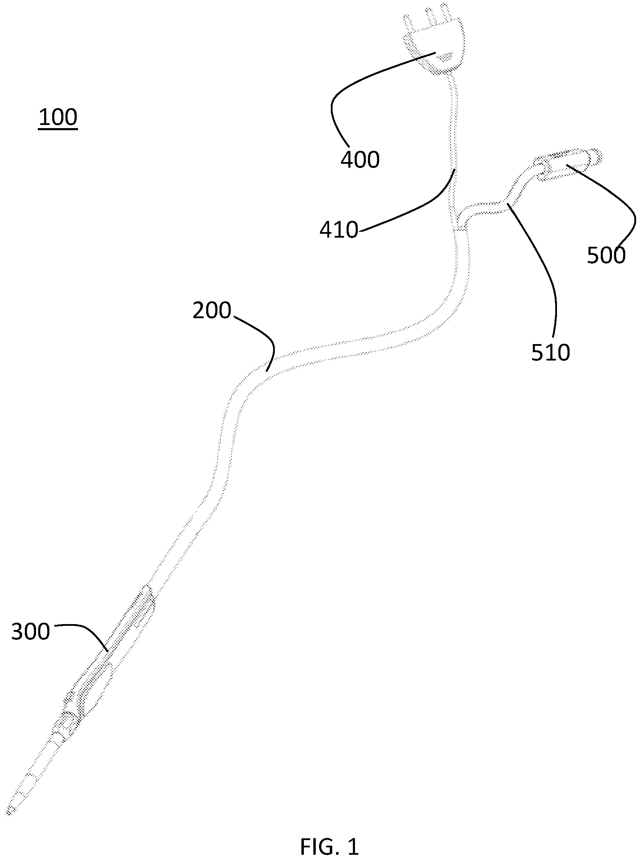

As shown in FIG. 1, a cable in accordance with the present invention can be used in an electrosurgical system, which, for example, may be a cold plasma electrosurgical system, a hybrid plasma electrosurgical system, or an argon coagulation electrosurgical system. The cable 200 of the present invention may have an electrical connector 400 and a gas supply connector 500 on one end and a handpice 300 on its other end. The electrical connector will have wiring 410 from the cable 200 and the gas connector will have a tube 510 from the cable 200. Various known connectors 400 and 500 may be used with the present invention.

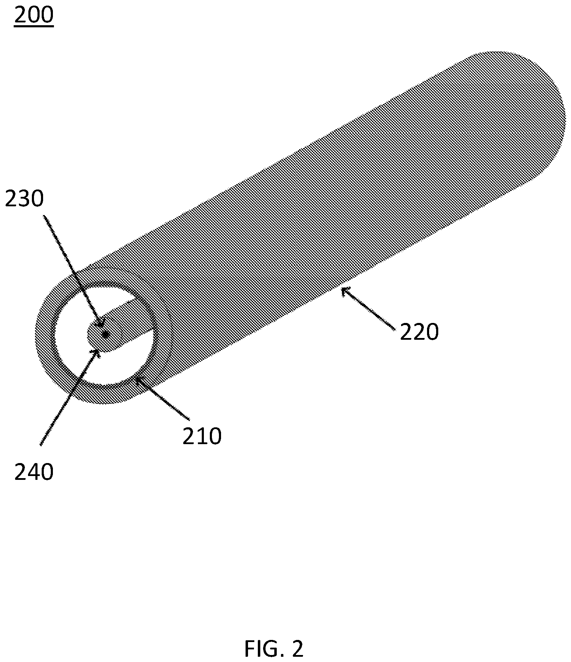

As shown in FIG. 2, the cable 200 has an inner electrode 230 to be connected to an electrosurgical generator, surrounded by insulation 240. When the cable is in use, this inner electrode 230 would be connected to a power supply through connector 400. In the preferred embodiment, the electrode 230 is made of cylindrical stainless steel wires of 0.25 mm radius embedded in silicon rubber insulator with radius about 2.5 mm. Material and diameter of the wire is not limited to utilization of stainless steel and other electrically conducting materials can be used as well. Preferentially, diameter (a) of the wire 230 should be chosen depending on precise maximal current requirements of the specific electrosurgical system. Radius (b) of insulator 240 and its material can be varied in wide range as well. In the preferred embodiment silicon rubber was used as material for insulator 240 having relative dielectric permittivity $\varepsilon \sim 3$, however dielectrics with other values of $\varepsilon$ can be utilized as well. Preferentially, flexible electrically insulating material should be used to provide electrical insulation along with good flexibility of the electrosurgical cable as a whole.

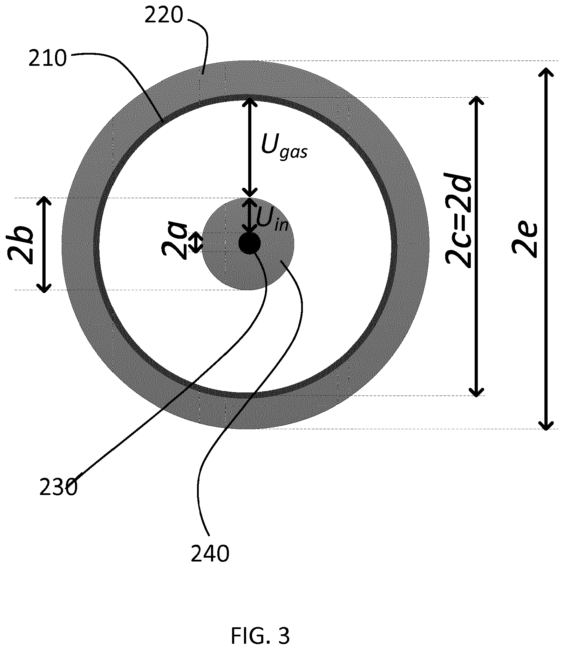

The cable further has an outer electrode 210 to be connected to a ground, surrounded on its exterior by electrical insulator 220. As shown in FIGs. 2-3, the outer conductor is cylindrical and forms a tube within which the inner conductor and inner insulation are placed such that a fluid channel is formed between the outer conductor 210 and the inner insulator 240. In the preferred embodiment, the outer electrode 210 is made of stainless steel braided sleeving embedded into outer insulating tube 220. Transparency of the braided shield can be varied depending on requirements of maximal cable weight. Lighter electrosurgical cables can be obtained by reducing diameter of the wire used in the braid and increasing of its transparency. Thin foil or other form of outer conductor can be used as well. Minimal cross-section of the outer conductor 210 should be limited by maximal electric current values required to be drawn through the particular electrosurgical cable. Inner radius (c) of the outer conductor 210 and outer radiuses (e) of insulator 220 and its material can be varied. Preferentially, flexible electrically insulating material should be used to provide electrical insulation along with good flexibility of the electrosurgical cable. The braided shield can be embedded inside the outer conductor and can have radius (d) in the range $c \leq d \leq e$. Note, Figure 3 shows the case when inner diameter of the outer conductor 210 is shown to be exactly equal to diameter of outer conductor 220 ($c$=$d$) and inner electrode 230 and the outer tube are coaxial.

In a preferred disclosure the inner conductor and outer conductor are cylindrical but other shapes may be sued with the invention.

In preferred disclosure Helium was used as working gas while other gases such as Argon can be used as well.

Relative sizes of the conductors 210, 230 and insulators 220, 240 should be chosen so that $a<b<c \leq d \leq e$. In the invention it was chosen $a$=0.25 mm, $b$=2.5 mm, $c$=$d$=4 mm and $e$=5 mm.

The total applied voltage ($U_0$) is distributed between the inner insulator ($U_{in}$) and gas gap between inner and outer insulators ($U_{gas}$), so that $U_0 = U_{in} + U_{gas}$ as shown in Figure 2. Ratio of voltages $U_{in}$ and $U_{gas}$, can be expressed as:

$$\frac{U_{in}}{U_{gas}} = \frac{\ln\frac{b}{a}}{\varepsilon \ln\frac{c}{b}}$$

In the invention the ratio $\frac{U_{in}}{U_{gas}}=1.1$ meaning that $U_{in} \approx U_{gas}$ and thus using $U_0 = U_{in} + U_{gas}$ one can obtain the that $U_{in} \approx U_{gas} \approx \frac{U_0}{2}$. In preferred embodiment, $U_0 \leq 4$kV was used and $U_{BD}$ was about 2.5 kV. Therefore, $U_{in} \approx U_{gas} \leq 2$ kV and thus $U_{gas} < U_{BD}$ providing that breakdown inside the electrosurgical cable prohibited. At the same time, $U_0 > U_{BD}$ and thus the voltage is sufficient to produce breakdown at the surgical handpiece. Various combinations of radiuses and dielectric permittivity can be used, however, it is critical to choose theses parameters so that two conditions are simultaneously satisfied:

1. $U_0 > U_{BD}$ - voltage is sufficient to produce breakdown at the surgical handpiece
2. $U_{gas} < U_{BD}$ - breakdown inside the electrosurgical cable is prohibited

In the preferred disclosure the inner electrode with insulator was freely placed inside the outer tube. However, relative location of the inner electrode with insulator with respect to the outer tube could be different such as coaxial or any other relative positioning. Also, inner insulator can be either permanently attached or not attached to the inner wall of the outer insulator.

The embodiment was chosen and described in order to explain the principles of the invention and its practical application to enable one skilled in the art to utilize the invention in various embodiments as are suited to the particular use contemplated. It is intended that the scope of the invention be defined by the claims appended hereto.

## Claims

1. An electrosurgical cable, on one end connecting to an electrosurgical power supply and a fluid source source and on the other end to a surgical instrument, comprising:

   an elongated outer conductor having an outer radius c;
   an outer insulator surrounding said outer conductor and having inner radius d and an outer radius e, said outer conductor and said outer insulator forming a tube;
   an elongated inner conductor inside said tube, said inner electrode having a radius a; and
   an inner insulator surrounding said inner conductor, said inner insulator having an outer radius b;
   wherein:

   there is a channel between an interior surface of said tube and said inner insulator,
   the radii a, b, c, d, e are selected so $a<b<c\leq d\leq e$
   **characterized in that**
   the radii a, b, c, d and e are selected so a total applied voltage ($U_0$) is distributed between the inner insulator ($U_{in}$) and gas gap between inner and outer insulators ($U_{gas}$), so that the applied voltage $U_0$ is greater than the breakdown voltage $U_{bd}$, $U_{gas}<U_{bd}$ and $U_0 = U_{in} + U_{gas}$,
   wherein a, b, c, d and e are selected so that $U_{in} \approx U_{gas}$, and
   $a=0.25$ mm, $b=2.5$ mm, $c=d=4$ mm and $e=5$ mm.

2. An electrosurgical cable according to claim 1, further comprising:

   an electrical connector connected to said inner conductor for connecting said inner conductor to an electrosurgical power supply;
   an electrical connector connected to said outer conductor for connecting said outer conductor to a ground; and
   a fluid connector connected to said tube for connecting said tube to a fluid source.

## Patentansprüche

1. Ein elektrochirurgisches Kabel, das an einem Ende mit einer elektrochirurgischen Stromversorgung und einer Flüssigkeitsquelle und an dem anderen Ende mit einem chirurgischen Instrument verbunden ist, umfassend:

   einen langgestreckten äußeren Leiter mit einem Außenradius c;
   einen äußeren Isolator, der den äußeren Leiter umgibt und einen Innenradius d und einen Außenradius e aufweist, wobei der äußere Leiter und der äußere Isolator ein Rohr bilden;
   einen langgestreckten inneren Leiter innerhalb des Rohrs, wobei der innere Leiter einen Radius a aufweist;
und
einen inneren Isolator, der den inneren Leiter umgibt, wobei der innere Isolator einen Außenradius b hat;
wobei:

ein Kanal zwischen einer Innenfläche des Rohrs und dem inneren Isolator vorhanden ist,
die Radien a, b, c, d, e sind so gewählt, dass a<b<c<=d<=e
**dadurch gekennzeichnet, dass**
die Radien a, b, c, d und e so gewählt sind, dass eine angelegte Gesamtspannung ($U_0$) verteilt ist
zwischen dem inneren Isolator ($U_{in}$) und dem Gasspalt zwischen innerem und äußerem Isolator ($U_{gas}$) verteilt ist, so daß die angelegte Spannung $U_0$ größer ist als die Durchschlagsspannung $U_{bd}$, $U_{gas}<U_{ba}$ und $U_0=U_{in} +U_{gas}$,
wobei a, b, c, d und e so gewählt sind, dass $U_{in}≈U_{gas}$ ist und a=0,25 mm, b=2,5 mm, c=d=4 mm und e=5 mm.

2. Elektrochirurgisches Kabel nach Anspruch 1, das ferner Folgendes umfasst:

einen elektrischen Verbinder, der mit dem inneren Leiter verbunden ist, um den inneren Leiter mit einer elektrochirurgischen Stromversorgung zu verbinden;
einen elektrischen Verbinder, der mit dem äußeren Leiter verbunden ist, um den äußeren Leiter mit der Erde zu verbinden; und
einen Fluidverbinder, der mit dem Rohr verbunden ist, um das Rohr mit einer Fluidquelle zu verbinden.

**Revendications**

1. Câble électrochirurgical, se connectant à une extrémité à une source d'alimentation électrochirurgicale et une source de fluide, et à l'autre extrémité à un instrument chirurgical, comprenant :

un conducteur extérieur allongé présentant un rayon extérieur *c* ;
un isolant extérieur entourant ledit conducteur extérieur et présentant un rayon intérieur *d* et un rayon extérieur *e,* ledit conducteur extérieur et ledit isolant extérieur formant un tube ;
un conducteur intérieur allongé à l'intérieur dudit tube, ledit conducteur intérieur présentant un rayon *a ;* et
un isolant intérieur entourant ledit conducteur intérieur, ledit isolant intérieur présentant un rayon extérieur *b* ;
selon lequel :

un canal est présent entre une surface intérieure dudit tube et dudit isolant intérieur,
les rayons *a, b, c, d, e* sont choisis de sorte que $a<b<c≤d≤e$
**caractérisé en ce que**
les rayons *a, b, c, d* et *e* sont choisis de sorte qu'une tension appliquée totale ($U_0$) est distribuée entre l'isolant intérieur ($U_{in}$) et l'espace à gaz entre les isolants intérieur et extérieur ($U_{gas}$), de sorte que la tension appliquée $U_0$ est supérieure à la tension de claquage $U_{bd}$, $U_{gas}<U_{bd}$ et $U_0 = U_{in} + U_{gas}$, selon lequel *a, b, c, d* et *e* sont choisis de sorte que $U_{in}≈U_{gas}$, et
*a* = 0.25 mm, *b* = 2.5 mm, *c*=*d*=4 mm et *e* = 5 mm.

2. Câble électrochirurgical selon la revendication 1, comprenant en outre :

un connecteur électrique connecté au dit conducteur intérieur pour connecter ledit conducteur intérieur à une source d'alimentation électrochirurgicale ;
un connecteur électrique connecté au dit conducteur extérieur pour connecter ledit conducteur extérieur à une mise à la terre ; et
un connecteur à fluide connecté au dit tube pour connecter ledit tube à une source de fluide.

EP 3 363 026 B1

100

400

410

200

500

510

300

FIG. 1

6

FIG. 2

FIG. 3

**EP 3 363 026 B1**

## REFERENCES CITED IN THE DESCRIPTION

### Patent documents cited in the description

- GB 2052836 A

- EP 1832243 A